(19) Europäisches Patentamt
European Patent Office
Office européen des brevets

(11) Publication number: **0 517 301 A1**

(12) **EUROPEAN PATENT APPLICATION**

(21) Application number: **92201504.5**

(22) Date of filing: **25.05.92**

(51) Int. Cl.5: **C07C 323/62**, A01N 37/38, C07C 323/63, C07C 323/65, C07D 239/26, C07D 249/08, C07D 271/06, C07D 277/10, C07D 277/26, A01N 37/50, A01N 43/00

(30) Priority: **05.06.91 GB 9112038**
**05.06.91 GB 9112039**
**05.06.91 GB 9112099**
**05.06.91 GB 9112110**

(43) Date of publication of application:
**09.12.92 Bulletin 92/50**

(84) Designated Contracting States:
**PT**

(71) Applicant: **SCHERING AGROCHEMICALS LIMITED**

**Hauxton Cambridge CB2 5HU(GB)**

(72) Inventor: **Richards, Ian Christopher**
**c/o Chesterford Park Research Station**
**Saffron Walden, Essex CB10 1XL(GB)**
Inventor: **Simpson, Donald James**
**c/o Chesterford Park Research Station**
**Saffron Walden, Essex CB10 1XL(GB)**
Inventor: **Whalley, Anthony Edward**
**c/o Chesterford Park Research Station**
**Saffron Walden, Essex CB10 1XL(GB)**

(74) Representative: **Waldman, Ralph David et al**
**Schering Agrochemicals Limited Industrial Property Department Chesterford Park Research Station**
**Saffron Walden Essex CB10 1XL(GB)**

(54) **Propenoic acid derivatives.**

(57) Compounds of formula I

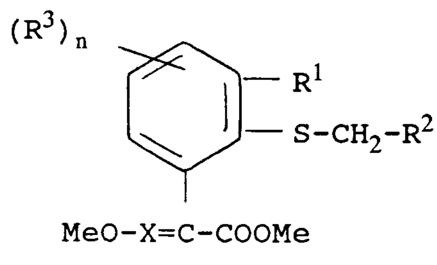

(I)

wherein X is CH or N; n is 0, 1, 2 or 3;
$R^1$ and $R^3$, which may be the same or different, are alkyl, alkoxy or alkylthio, each of which is optionally substituted, halogen, nitro, cyano, $COOR^4$, $-NR^5R^6$, $CONR^5R^6$, $COR^7$ or $R^8S(O)_q$; or $R^1$ and an adjacent $R^3$ group, or two adjacent $R^3$ groups, together with the carbon atoms to which they are attached can form a 5 to 8 membered ring which can include 1 to 3 heteroatoms and may be substituted;
$R^2$ is an optionally substituted aliphatic hydrocarbon radical, which may be unsaturated, aryl or heterocyclyl;
$R^4$ is hydrogen or an ester forming group;
$R^5$ and $R^6$ are the same or different and are hydrogen, optionally substituted alkyl, acyl or aryl, or together with the nitrogen to which they are attached, form a 5 to 7 membered ring which can contain other hetero atoms;

EP 0 517 301 A1

$R^7$ is hydrogen, optionally substituted alkyl or aryl;
$R^8$ is optionally substituted alkyl or aryl; and
q is 1 or 2, have fungicidal activity as do the intermediates of formula II

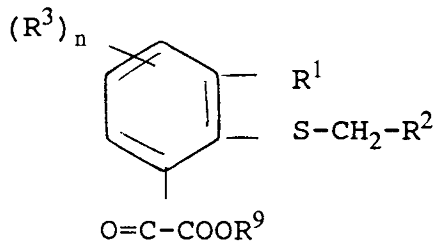

(II)

wherein $R^9$ is hydrogen or methyl, and of formula III

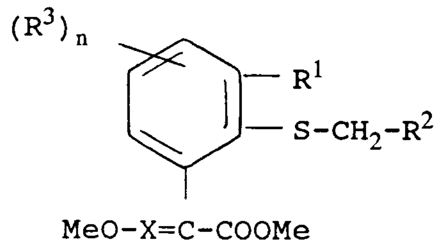

(I)

## Field of the invention

This invention relates to propenoic acid derivatives, having fungicidal activity.

## Prior Art

Alkyl 2-aryl-2-propenoate derivatives have been disclosed as having pesticidal activity. In our EP 299694, we have disclosed compounds of this type and in particular 2-phenyl-2-propenoic acid derivatives in which the phenyl group is substituted in the 2-position by substituted thio groups. Analogous compounds have also been disclosed in EP 251082. Although many of these compounds have outstanding fungicidal activity, some compounds of this type appear to be unstable when exposed to light and the activity is thus not always maintained over a long period.

## Description of the Invention

We have now found that introduction of certain substituents in the 3-position of the aryl ring results in compounds having good fungicidal activity coupled with greater stability.

Thus according to one aspect of the invention there is provided a compound of formula I

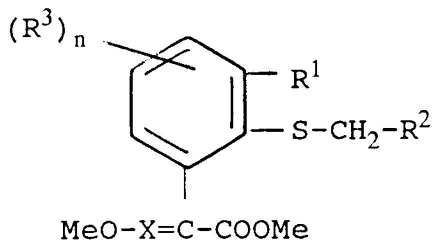

(I)

wherein

- X is CH or N;
- n is 0, 1, 2 or 3;
- $R^1$ and $R^3$, which may be the same or different, are alkyl, alkoxy or alkylthio, each of which is optionally substituted, halogen, nitro, cyano, $COOR^4$, $-NR^5R^6$,$CONR^5R^6$, $COR^7$ or $R^8S(O)_q$; or $R^1$ and an adjacent $R^3$ group, or two adjacent $R^3$ groups, together with the carbon atoms to which they are attached can form a 5 to 8 membered ring which can include 1 to 3 heteroatoms and may be substituted;
- $R^2$ is an optionally substituted aliphatic hydrocarbon radical, which may be unsaturated, aryl or heterocyclyl;
- $R^4$ is hydrogen or an ester forming group;
- $R^5$ and $R^6$ are the same or different and are hydrogen, optionally substituted alkyl, acyl or aryl, or together with the nitrogen to which they are attached, form a 5 to 7 membered ring which can contain other hetero atoms;
- $R^7$ is hydrogen, optionally substituted alkyl or aryl;
- $R^8$ is optionally substituted alkyl or aryl; and
- q is 1 or 2.

Aliphatic hydrocarbon radicals are generally of up 10 carbon atoms and can be cyclic or acyclic. Alkyl groups may branched or straight chained. If $R^2$ is alkyl it is generally of 4 to 9 carbon atoms. Substituents, when present on any aliphatic hydrocarbon radical group, include halogen, alkoxy (e.g. of 1 to 4 carbon atoms), haloalkoxy (e.g. difluoromethoxy), hydroxy, alkylthio, nitro, optionally substituted amino, carboxy, alkoxycarbonyl, cyano, acyloxy and aryl. Cyclic aliphatic groups are generally of 3 to 8 carbon atoms. Aryl groups are usually phenyl, optionally substituted, e.g. by halogen, optionally substituted alkyl or alkoxy, aryl, aryloxy, nitro, amino, COOH, alkoxycarbonyl, CN, $CONR^5R^6$ or $S(O)_pR^8$ (p = 0, 1 or 2). In some cases two substituents, together with the phenyl to which they are attached, can form a fused ring which itself can be optionally substituted as for phenyl. The terms heterocyclyl includes aromatic and non-aromatic rings which usually contain 5 to 7 ring atoms and including up to three hetero atoms usually selected from nitrogen, oxygen and sulfur. Examples of such groups include thienyl, furyl, pyridyl, pyrimidinyl, pyrazolyl, thiazolyl, thiazolinyl, thiazolone, oxazolyl, benzimidazolyl, tetrazolyl, benzoxazolyl, thiadiazolyl, dioxolanyl, imidazopyridinyl, 1,3-benzoxazinyl, 1,3-benzothiazinyl, oxazolopyridinyl, triazolyl, triazinyl, imidazolyl, morpholino, benzofuranyl, pyrazolinyl, quinolinyl, quinazolinyl, dihydroquinazolinyl or benzothiazolyl. The heterocyclyl group may be substituted, e.g. as described for phenyl. The term "acyl" includes the residue of sulfonic and phosphorus containing acids as well as carboxylic acids. Amino groups may be substituted, e.g. by one or two alkyl groups or two substituents can form a ring, e.g. to form a morpholino or piperidino ring. In the compounds of the invention, alkyl groups, which may be branched or straight chains, are preferably of 1 to 8 carbon atoms. $R^1$ is generally of 1 to 4 carbon atoms, especially methyl or ethyl. Alkenyl and alkynyl groups are generally of three to ten carbon atoms.

It is generally preferred that n is 0 or 1, especially 0.

$R^1$ is preferably methyl, ethyl, methoxy, ethoxy or halogen, e.g. chlorine or bromine. It is particularly preferred that $R^1$ is methyl, methoxy or chlorine.

$R^2$ is preferably heteroaryl or phenyl, optionally substituted by one or more of the same or different halogen, alkyl, haloalkyl, $C_{3-8}$-cycloalkyl, alkoxy, haloalkoxy, cyanoalkoxy, alkylthio, alkanoyl, alkoxycarbonyl, alkoxyalkoxy, optionally substituted phenyl, optionally substituted phenylalkoxy, optionally substituted phenylalkyl, optionally substituted phenoxyalkoxy, alkenyl, alkenyloxy, alkynyl, alkynyloxy, alkylsulfonyl, haloalkylsulfonyl, alkoxycarbonylalkyl, alkoxyiminoalkyl, cyano, nitro, dialkylaminoalkyl or heterocyclyl, in which substituents on any optionally substituted phenyl group are halogen, alkyl, haloalkyl, alkoxy, nitro or cyano, any aliphatic group is of 1 to 8 carbon atoms and any heteroaryl group comprises 5 or 6 ring atoms including up to 3 hetero atoms selected from nitrogen, sulfur and oxygen, and any heterocyclyl substituent is itself optionally substituted by alkyl, halo or optionally substituted phenyl.

Compounds of the invention exist as structural isomers and the invention includes individual isomers as well as mixtures of these. Preferred compounds are those where the methoxypropenoate or (methoxyimino)acetate attached directly to the benzene ring shown in formula I is in the E-configuration.

The compounds of formula I have pesticidal activity and especially fungicidal activity. This activity manifests itself especially against fungal diseases of plants, e.g. mildews and particularly barley powdery mildew (Erysiphe graminis) and vine downy mildew (Plasmopara viticola), rice blast (Pyricularia oryzae), cereal eyespot (Pseudocercosporella herpotrichoides), grey mould (Botrytis cinerea), wheat brown rust (Puccinia recondita), late tomato or potato blight (Phytophthora infestans), apple scab (Venturia inaequalis), rice sheath blight (Pellicularia sasakii), glume blotch (Lentosphaeria nodorum). Other fungi against which the compounds may be active include other powdery mildews, other rusts, and general pathogens of Deuteromycete, Ascomycete, Phycomycete and Basidiomycete origin.

Some compounds of the invention also have insecticidal, acaricidal and nematicidal activity and are particularly useful in combating a variety of economically important insects, acarids and plant nematodes, including animal ectoparasites. They may also have herbicidal activity.

The invention thus also provides a method of combating pests (i.e. fungi, insects, nematodes, acarids and weeds, but especially fungi) at a locus infested or liable to be infested therewith, which comprises applying to the locus a compound of formula I.

The invention also provides an agricultural composition comprising a compound of formula I in admixture with an agriculturally acceptable diluent or carrier.

The composition of the invention may of course include more than one compound of the invention.

In addition the composition can comprise one or more additional active ingredients, for example compounds known to possess plant-growth regulant, herbicidal, fungicidal, insecticidal or acaricidal properties. Alternatively the compounds of the invention can be used in sequence with the other active ingredient.

The diluent or carrier in the composition of the invention can be a solid or a liquid optionally in association with a surface-active agent, for example a dispersing agent, emulsifying agent or wetting agent. Suitable surface-active agents include anionic compounds such as a carboxylate, for example a metal carboxylate of a long chain fatty acid; an N-acylsarcosinate; mono- or di-esters of phosphoric acid with fatty alcohol ethoxylates or salts of such esters; fatty alcohol sulfates such as sodium dodecyl sulfate, sodium octadecyl sulfate or sodium cetyl sulfate; ethoxylated fatty alcohol sulfates; ethoxylated alkylphenol sulfates; lignin sulfonates; petroleum sulfonates; alkyl-aryl sulfonates such as alkyl-benzene sulfonates or lower alkylnaphthalene sulfonates, e.g. butyl-naphthalene sulfonate; salts of sulfonated naphthalene-formaldehyde condensates; salts of sulfonated phenol-formaldehyde condensates; or more complex sulfonates such as the amide sulfonates, e.g. the sulfonated condensation product of oleic acid and N-methyl taurine or the dialkyl sulfosuccinates, e.g. the sodium sulfonate of dioctyl succinate. Nonionic agents include condensation products of fatty acid esters, fatty alcohols, fatty acid amides or fatty-alkyl- or alkenyl-substituted phenols with ethylene oxide, fatty esters of polyhydric alcohol ethers, e.g. sorbitan fatty acid esters, condensation products of such esters with ethylene oxide, e.g. polyoxyethylene sorbitan fatty acid esters, block

copolymers of ethylene oxide and propylene oxide, acetylenic glycols such as 2,4,7,9-tetramethyl-5-decyne-4,7-diol, or ethoxylated acetylenic glycols.

Examples of a cationic surface-active agent include, for instance, an aliphatic mono-, di-, or polyamine as an acetate, naphthenate or oleate; an oxygen-containing amine such as an amine oxide or polyoxyethylene alkylamine; an amide-linked amine prepared by the condensation of a carboxylic acid with a di- or polyamine; or a quaternary ammonium salt.

The compositions of the invention can take any form known in the art for the formulation of agrochemicals, for example, a solution, a dispersion, an aqueous emulsion, a dusting powder, a seed dressing, a fumigant, a smoke, a bait, a dispersible powder or granule, an emulsifiable concentrate or granules. Moreover it can be in a suitable form for direct application or as a concentrate or primary composition which requires dilution with a suitable quantity of water or other diluent before application.

An emulsifiable concentrate comprises a compound of the invention dissolved in a water-immiscible solvent which is formed into an emulsion with water in the presence of an emulsifying agent.

A dusting powder comprises a compound of the invention intimately mixed and ground with a solid pulverulent diluent, for example, kaolin.

A granular solid comprises a compound of the invention associated with similar diluents to those which may be employed in dusting powders, but the mixture is granulated by known methods. Alternatively it comprises the active ingredient absorbed or adsorbed on a pre-granular diluent, for example, Fuller's earth, attapulgite or limestone grit.

Wettable powders, granules or grains usually comprise the active ingredient in admixture with a suitable surfactant and an inert powder diluent such as china clay.

Another suitable concentrate is a flowable suspension concentrate which is formed by grinding the compound with water or other liquid, a wetting agent and a suspending agent.

The concentration of the active ingredient in the composition of the present invention, as applied to plants is preferably within the range of 0.0001 to 3.0 per cent by weight, especially 0.001 to 0.1 per cent by weight. In a primary composition the amount of active ingredient can vary widely and can be, for example, from 5 to 95 per cent by weight of the composition.

In the method of the invention the compound is generally applied to seeds, plants or their habitat. Thus the compound can be applied directly to the soil before, at or after drilling so that the presence of active compound in the soil can control the growth of fungi which may attack seeds. When the soil is treated directly the active compound can be applied in any manner which allows it to be intimately mixed with the soil such as by spraying, by broadcasting a solid form of granules, or by applying the active ingredient at the same time as drilling by inserting it in the same drill as the seeds. A suitable application rate is within the range of from 0.05 to 20 kg per hectare, more preferably from 0.1 to 10 kg per hectare.

Alternatively the active compound can be applied directly to the plant by, for example, spraying or dusting either at the time when the fungus has begun to appear on the plant or before the appearance of fungus as a protective measure. In both such cases the preferred mode of application is by foliar spraying. It is generally important to obtain good control of fungi in the early stages of plant growth as this is the time when the plant can be most severely damaged. The spray or dust can conveniently contain an insecticide if this is thought necessary. Sometimes, it is practicable to treat the roots of a plant before or during planting, for example, by dipping the roots in a suitable liquid or solid composition. When the active compound is applied directly to the plant a suitable rate of application is from 0.001 to 5 kg per hectare, preferably from 0.01 to 0.5 kg per hectare.

The compounds of the invention may be obtained by various methods. For example when X is CH, a compound of formula II

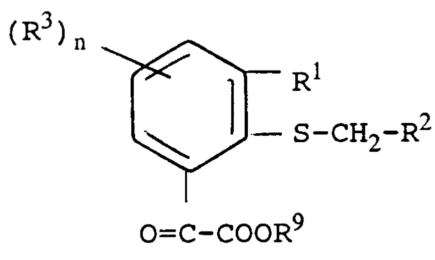

(II)

where $R^9$ is methyl, can be reacted with a phosphorus ylide derived from a phosphonium salt of formula

$Ph_3P^+CH_2OMe\ Hal^-$

under basic conditions.

When X is N, the compound of formula II can be reacted with methoxyamine hydrochloride.

Alternatively when X is CH, a compound of formula III

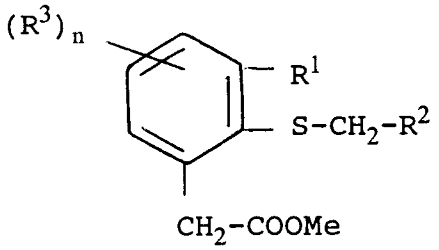

(III)

can be reacted with methyl formate under basic conditions, followed by methylation of the resulting hydroxypropenoate.

The compounds of formula II, where $R^9$ is hydrogen, can be obtained by reacting a benzo[b]thiophene-2,3-dione of formula IV

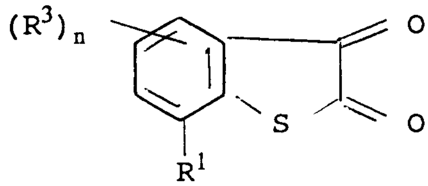

(IV)

with a compound of formula V

$R^2$-$CH_2$-Z (V)

where Z is a leaving group, e.g. halogen, generally under basic conditions. This can then be esterified to a compound of formula II, where $R^9$ is methyl, e.g. by reacting with methyl chloroformate in the presence of a tertiary amine.

The compounds of formula III can be obtained by reacting a benzo[b]thiophen-2(3H)-one of formula VI

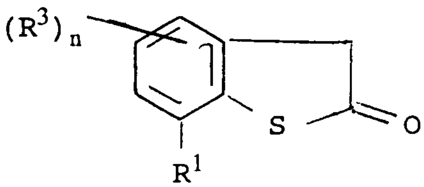

(VI)

with a compound of formula V

$R^2$-$CH_2$-Z (V)

where Z is a leaving group, eg halogen, generally under basic conditions, followed by esterification in conventional manner.

The compounds of formula III can also be obtained by reducing the compounds of formula II, preferably where where $R^9$ is hydrogen, in a Wolff-Kischner reduction, e.g. by using hydrazine in the presence of a base e.g. an alkali metal alkoxide. When $R^9$ is hydrogen, the Wolff-Kischner reduction is followed by esterification to the methyl ester.

The compounds of formulae IV, V and VI are known or can be obtained in known manner.

The compounds of formula II and III are novel and the invention also includes these compounds of this type. Many of these compounds also have pesticidal activity.

The compounds of the invention may also be obtained by the method described in our PCT application WO 91/07385.

The invention is illustrated in the following Examples. Structures of isolated novel compounds were confirmed by elemental and/or other appropriate analyses. Temperatures are in °C.

Example 1

A mixture of 7-methylbenzo[b]thiophene-2,3-dione (4.1 g) and aqueous sodium hydroxide (1.84 g in 80 ml water) was heated under reflux for one hour. To the resulting solution at 50°, was added 4-chloro-3-methoxybenzyl bromide (5.42 g) and the mixture refluxed for a further 2 hours and allowed to cool. It was acidified to pH 1, extracted with ether and the extract dried and evaporated. The residue was recrystallised from diisopropyl ether to give 2-[2-(4-chloro-3-methoxybenzylthio)-3-methylphenyl]-2-oxoacetic acid, m.p. 117-8°. (Intermediate A)

To a solution of Intermediate A (5.8 g) in dichloromethane (100 ml) was added triethylamine (1.84 g), followed by dropwise addition of methyl chloroformate (1.64 g). The mixture was heated under reflux for 0.5 hours, cooled, washed with aqueous sodium hydrogen carbonate, dried and evaporated. The residue was recrystallised from diisopropyl ether to give methyl 2-[2-(4-chloro-3-methoxy-benzylthio)-3-methylphenyl]-2-oxoacetate, m.p. 118-9° (Intermediate B).

A solution of Intermediate B (4.2 g) in ether (60 ml) was added dropwise to a mixture of potassium tert butoxide (2.6 g) and a suspension of (methoxymethyl)triphenyl-phosphonium chloride (7.9 g) in ether (100 ml), which had been stirred for 45 minutes. The reaction mixture was stirred for 4 hours, washed with water, dried and evaporated. The residue was dissolved in dichloromethane, filtered through silica gel and evaporated. The residue was recrystallised from diisopropyl ether to give methyl (E)-3-methoxy-2-[2-(4-chloro-3-methoxybenzylthio)-3-methylphenyl]prop-2-enoate, m.p. 100-2°. (Compound 1)

In a similar manner the following compounds of formula I, and their type A and B intermediates were obtained. Where no parameters are given for an intermediate then it was not isolated in a pure form but used in its crude form in the next reaction stage. Generally the compounds are obtained as E-isomers; compounds obtained as Z-isomers are indicated by an asterisk (*), and are obtained from the reaction mixture by chromatography on silica gel using ethyl acetate/petoleum ether as eluent.

| Cpd. No. | $R^1$ | $R^2$ | $(R^3)_n$ | m.p.(°) or physical state: Compound of formula I | Intermediates A | B |
|---|---|---|---|---|---|---|
| 2 | Me | 3-Me-Ph | - | 141-3 | 131-3 | oil |
| 3 | Me | 4-$CF_3$-Ph | - | 103-5 | 105-7 | 70-2 |
| 4 | Me | 3-MeO-Ph | - | 89-90 | 73-5 | 55-7 |
| 5 | Me | 3-F-Ph | - | 110-2 | 122-4 | 55-7 |
| 6 | Me | 2-Br-Ph | - | 130-1 | 141-3 | 68-70 |
| 7 | Me | 2-Cl-Ph | - | 100-2 | 105-7 | 68-70 |

| Cpd. No. | $R^1$ | $R^2$ | $(R^3)_n$ | m.p.(°) or physical state Compound of formula I | Intermediates A | B |
|---|---|---|---|---|---|---|
| 8 | Me | 4-Et-Ph | - | 68-70 | 84-5 | oil |
| 9 | Me | 3,5-$Me_2$-Ph | - | 120-2 | 142-4 | 68-70 |
| 10 | Me | 2,5-$Me_2$-Ph | - | 132-3 | 110-2 | 82-3 |
| 11 | Me | Ph | - | 120-2 | 103-5 | 44-5 |
| 12 | Me | 2-($PhCH_2CH_2$)-Ph | - | 75-7 | 107-9 | oil |
| 13 | Me | 2,5-$(CF_3)_2$-Ph | - | oil* | 75-6 | 80-1 |
| 14 | Me | 3,5-$Cl_2$-Ph | - | 130-1 | 131-3 | 101-3 |
| 15 | Me | 3-(4-Cl-Ph)-1,2,4-triazol-1-yl | - | 107-9 | 197-9 | 95-6 |
| 16 | Et | 4-Me-Ph | - | 92-3 | 79-80 | oil |
| 17 | Me | 4-F-3-Me-Ph | - | 142-3 | 112-4 | 73-4 |
| 18 | Me | 3-MeO-Ph | - | 83-5* | 73-5 | 55-7 |
| 19 | Me | Ph | - | 103-5* | 103-5 | 44-5 |
| 20 | Me | 3-($PhCH_2O$)-Ph | - | 102-4 | 98-100 | 88-90 |
| 21 | Me | 3-EtO-Ph | - | 80-1 | 110-2 | oil |
| 22 | Me | 3-($C_5H_{11}O$)-Ph | - | 78-80 | | oil |
| 23 | Me | 3-($C_6H_{13}OCO$)-Ph | - | oil* | | oil |
| 24 | Me | 3-($C_6H_{13}OCO$)-Ph | - | 74-6 | | oil |
| 25 | Me | 2,5-$(CF_3)_2$-Ph | - | 77-8 | 75-6 | 80-1 |
| 26 | Cl | 3-Me-Ph | - | 101-2 | 133-4 | 80-1 |
| 27 | Me | 4-($CF_3O$)-Ph | - | 79-80 | 116-7 | oil |
| 28 | Me | 4-($CF_3O$)-Ph | - | 87-8* | 116-7 | oil |
| 29 | Me | 4-Cl-Ph | - | 95-7* | 135-7 | 68-70 |
| 30 | Me | 3-(3-Me-Ph)-1,2,4-triazol-1-yl | - | gum | | oil |
| 31 | Me | 4-MeCONH-Ph | - | 70-2 | | 130-2 |
| 32 | Et | 3-F-Ph | - | 96-8 | 97-9 | 73-5 |
| 33 | Me | 4-Cl-Ph | - | 115-7 | 135-7 | 68-70 |
| 34 | Et | 3-MeO-Ph | - | oil | 74-4.5 | 51.5-2.5 |
| 35 | Et | 3-F-Ph | - | oil* | | 73-75 |

| Cpd. No. | $R^1$ | $R^2$ | $(R^3)_n$ | m.p.(°) or physical state Compound of formula I | Intermediates A | B |
|---|---|---|---|---|---|---|
| 36 | Me | 3-Bu-Ph | - | oil* | | oil |
| 37 | Et | 4-Cl-Ph | - | 86-7 | 89.5-90.5 | gum |
| 38 | Me | 2-$CF_3$-Ph | - | 84-6 | 123-4 | 73-4 |
| 39 | Me | 3-Bu-Ph | - | oil | | oil |
| 40 | Me | 2-$CF_3$-Ph | - | 76-7* | 123-4 | 73-4 |
| 41 | Et | 3,5-$Me_2$-Ph | - | 116.5-8.5 | 80-1 | 52-3.5 |
| 42 | Me | 3,5-$(MeO)_2$-Ph | - | 115-6 | 156-8 | 80-1 |
| 43 | Me | 3,5-$(MeO)_2$-Ph | - | oil* | 156-8 | 80-1 |
| 44 | Me | 3-$CF_3$-Ph | - | oil* | | |
| 45 | Et | 3-($C_5H_{11}$OCO)-Ph | - | oil | oil | oil |
| 46 | Et | 3-($C_5H_{11}$OCO)-Ph | - | oil* | oil | oil |
| 47 | Me | 2-Me-Ph | - | 105-6 | 123-4 | 45-6 |
| 48 | Me | 2-Me-Ph | - | 99-100* | 123-4 | 45-6 |
| 49 | Me | 2,5-$Cl_2$-Ph | - | 111-2 | 148-9 | 110-1 |
| 50 | Me | 2,5-$Cl_2$-Ph | - | 105-6* | 148-9 | 110-1 |
| 51 | Me | 3-$CF_3$O-Ph | - | 52.5-4 | 100.5-2 | oil |
| 52 | Me | 3,5-$F_2$-Ph | - | 86-7* | 117-8 | 63-4 |
| 53 | Me | 3-$CF_3$O-Ph | - | gum* | 100.5-2 | oil |
| 54 | Me | 2-$CF_3$O-Ph | - | 72.5-3.5 | 124-5 | 61.5-3 |
| 55 | Me | 3-$CF_3$-Ph | - | 90-1 | 103-4 | 72-3 |
| 56 | Me | 3-$CHF_2$O-5-Me-Ph | - | gum | 127-8.5 | oil |
| 57 | Me | 3-$CHF_2$O-5-Me-Ph | - | 93-4* | 127-8.5 | oil |
| 58 | Me | 3-Cl-5-Me-Ph | - | 132-3.5 | 150-1 | 98-9.5 |
| 59 | Me | 3-MeO-5-Me-Ph | - | 88-9 | 112-3 | 50-1.5 |
| 60 | Me | 3,5-$F_2$-Ph | - | 118-9 | 117-8 | 63-4 |
| 61 | Et | 3,5-$Cl_2$-Ph | - | 126-7 | 100-1 | 55-7 |
| 62 | Me | 3-MeOCO-Ph | - | oil | 115-6 | |
| 63 | Me | 3-Cl-Ph | - | 126-7 | 127-9 | 99-100.5 |
| 64 | Me | 3-MeO-5-Me-Ph | - | 74-5.5* | 112-3 | 50-1.5 |
| 65 | Et | 3,5-$Cl_2$-Ph | - | 118-9* | 100-1 | 56-7 |

| Cpd. No. | $R^1$ | $R^2$ | $(R^3)_n$ | m.p.(°) or physical state Compound of formula I | Intermediates A | B |
|---|---|---|---|---|---|---|
| 66 | Et | Ph | - | 61-2* | 108-10 | 43-4 |
| 67 | Me | 2-MeO-Ph | - | 108-9 | 121-2 | 83-4 |
| 68 | Me | 2-MeO-Ph | - | 112-3* | 121-2 | 83-4 |
| 69 | Et | Ph | - | 86-7 | 108-10 | 43-4 |
| 70 | Et | 4-Et-Ph | - | 60* | oil | 52-4 |
| 71 | Me | 3-($C_5H_{11}OCO$)-5-Me-Ph | - | oil | oil | oil |
| 72 | Me | 4-(4,6-$Me_2$-pyrimidin-2-yl)-Ph | - | 158-8.5 | 182.5-3.5 | 103.5-6 |
| 73 | Me | 3-(4,6-$Me_2$-pyrimidin-2-yl)-Ph | - | 128-129.5 | 209.5-212.5 | 109-111.5 |
| 74 | Me | 3-(4,6-$Me_2$-pyrimidin-2-yl)-Ph | - | 110.5-112.5* | 209.5-212.5 | 109.5-111.5 |
| 75 | Et | 4-Et-Ph | - | 87-9 | oil | 52-4 |
| 76 | Me | 4-MeO-Ph | - | 76-7 | 100-0.5 | oil |
| 77 | Me | 4-MeO-Ph | - | 108.5-9.5* | 100-0.5 | oil |
| 78 | Me | 2-Et-Ph | - | 98-9 | 105-6 | 54-5 |
| 79 | Me | 2-Et-Ph | - | 58-60* | 105-6 | 54-5 |
| 80 | Me | 2,5-$F_2$-Ph | - | 123-4* | 92-5 | 67-8 |
| 81 | Me | 3,4-$Me_2$-Ph | - | 129-31 | 118-20 | 55-6 |
| 82 | Me | 3-Et-Ph | - | 103-5 | | oil |
| 83 | Me | 3-($CH_2$=CH-)-Ph | - | 104-7 | 211 | oil |
| 84 | Me | 3-($CH_2$=CH-)-Ph | - | gum* | 211 | oil |
| 85 | Et | 3-($C_5H_{11}OCO$)-5-Me-Ph | - | oil* | oil | oil |
| 86 | Me | 3-($CHF_2O$)-Ph | - | 111-2* | 200(dec) | oil |
| 87 | Me | 3-(pyrimidin-2-yl)-Ph | - | 115-6* | 135-7 | 88-9 |
| 88 | Me | 3-($CHF_2O$)-Ph | - | gum | 200(dec) | oil |
| 89 | Me | 3-(pyrimidin-2-yl)-Ph | - | 110-2 | 135-7 | 88-9 |
| 90 | Me | 2,5-$F_2$-Ph | - | 104-6 | 92-5 | 67-8 |
| 91 | Me | 3-Et-Ph | - | 69.5-71* | | oil |
| 92 | Me | 2,4,5-$Me_3$-Ph | - | 169-70 | 148-9.5 | 66.5-66.8 |

| Cpd. No. | $R^1$ | $R^2$ | $(R^3)_n$ | m.p.(°) or physical state Compound of formula I | Intermediates A | B |
|---|---|---|---|---|---|---|
| 93 | Me | 2-F-5-$CF_3$-Ph | - | 78-80 | 93-5 | 100-2 |
| 94 | Me | 2-F-5-$CF_3$-Ph | - | 96-8* | 93-5 | 100-2 |
| 95 | Me | 2-F-6-$CF_3$-Ph | - | 80-2 | 125-6 | 73-5 |
| 96 | Me | 2-F-6-$CF_3$-Ph | - | 98-100* | 125-6 | 73-5 |
| 97 | Me | 4-F-3-$CF_3$-Ph | - | 82.5-4 | 140-1.5 | 82.5-3.5 |
| 98 | Me | 4-F-3-$CF_3$-Ph | - | 88-9* | 140-1.5 | 82.5-3.5 |
| 99 | Me | 3-F-5-$CF_3$-Ph | - | 87.5-8.5* | 97.5-8.5 | 50-0.5 |
| 100 | Me | 3-(1-Me-pentyl-OCO)-Ph | - | oil | | oil |
| 101 | Me | 4-(MeOCO)-Ph | - | 92-2.5 | 110.5-11 | 80-1 |
| 102 | Me | 4-(MeOCO)-Ph | - | 80-82.5* | 110.5-11 | 80-1 |
| 103 | Et | 4-(MeOCO)-Ph | - | 81.5-2* | 89.5-90.5 | 54-5.50 |
| 104 | Me | 4-(pyrimidin-2-yl)-Ph | - | 131-3* | 141-3 | 133-4 |
| 105 | Me | 4-(pyrimidin-2-yl)-Ph | - | 103-5 | 141-3 | 133-4 |
| 106 | Et | 3-cyano-Ph | - | 98.5-9.5 | 137.5-8.5 | 82.5-3.5 |
| 107 | Me | 3-$Pr^iO$-Ph | - | 65-6 | 87-88 | oil |
| 108 | Me | 3-F-Ph | - | 98-100* | 122-4 | 55-7 |
| 109 | Et | 4-(MeOCO)-Ph | - | 80-1 | 89.5-90.5 | 54-5.5 |
| 110 | Et | 3-MeO-Ph | - | 100.5-1.5* | 74-4.5 | 51.5-2.5 |
| 111 | Me | 2,4-$Me_2$-Ph | - | 114-6 | 121-22.5 | |
| 112 | Me | 3-F-5-Me-Ph | - | 127-8.5 | 124-6 | |
| 113 | Me | 3-F-5-Me-Ph | - | 109-110* | 124-6 | |
| 114 | Me | 4-($CHF_2O$)-Ph | - | 88.5-89 | oil | 61.5-2 |
| 115 | Me | 4-($CHF_2O$)-Ph | - | 89-91* | oil | 61.5-2 |
| 116 | Me | 3-I-Ph | - | 88-90 | 140-3 | 102-4 |
| 117 | Me | 3-I-Ph | - | 108-9.5* | 140-3 | 102-4 |
| 118 | Me | 3-Br-5-Me-Ph | - | 113-5 | 138-40 | 96-8 |
| 119 | Me | 3-Br-5-Me-Ph | - | 105-7* | 138-40 | 96-8 |
| 120 | Me | 3-$Pr^iO$-Ph | - | oil* | 87-88 | oil |
| 121 | Me | 2-Cl-5-MeS-Ph | - | 130-1 | 116-8 | 79-80 |
| 122 | Me | 2-Cl-5-MeS-Ph | - | 85-6* | 116-8 | 79-80 |

| Cpd. No. | $R^1$ | $R^2$ | $(R^3)_n$ | m.p.(°) or physical state Compound of formula I | Intermediates A | B |
|---|---|---|---|---|---|---|
| 123 | Me | 3-MeCOO-5-Me-Ph | - | oil | oil | 96-8 |
| 124 | Me | 4-(4-$NO_2$-$PhCH_2O$)-Ph | - | 82-3.5 | 127-8.5 | oil |
| 125 | Me | 3-Me-5-EtOCO-Ph | - | 89-90 | 103-4.5 | 67-8.5 |
| 126 | Me | 3-Me-5-EtOCO-Ph | - | oil* | 103-4.5 | 67-8.5 |
| 127 | Me | 3-MeS-Ph | - | 108-10 | 128-9 | oil |
| 128 | Me | 3-MeS-Ph | - | 65-6* | 128-9 | oil |
| 129 | Et | 2,5-$Cl_2$-Ph | - | 99-100 | 118-20 | 102-3 |
| 130 | Et | 2,5-$Cl_2$-Ph | - | oil* | 118-20 | 102-3 |
| 131 | Me | 3-F-5-$CF_3$-Ph | - | 42.5-4.5 | 97.5-8.5 | 50-50.5 |
| 132 | Et | 3-cyano-Ph | - | 98-9* | 137.5-8.5 | 82-3.5 |
| 133 | Et | 2,5-$Me_2$-Ph | - | 69.5-70.5* | 96.5-7.5 | oil |
| 134 | Et | 2,5-$Me_2$-Ph | - | 130-1 | 96.5-7.5 | oil |
| 135 | Me | 3-EtOCO-Ph | - | 76-8 | 93.5-4 | 48.5-50 |
| 136 | Me | 2-$MeOCOCH_2$-Ph | - | 101-3 | | 70-2 |
| 137 | Me | 4-Me-Ph | 5-Me | 142-3 | 123-4 | 82-3 |
| 138 | Me | 4-Me-Ph | 5-Me | 129-30* | 123-4 | 82-3 |
| 139 | Me | 3-MeO-Ph | 5-Me | 140-1 | 108-9 | oil |
| 140 | Me | 3-MeO-Ph | 5-Me | 82-3* | 108-9 | oil |
| 141 | Me | 2-F-Ph | 5-Me | 92-3 | 130-1 | 67-8 |
| 142 | Me | 2-F-Ph | 5-Me | 101-3* | 130-1 | 67-8 |
| 143 | Me | 4-Me-Ph | 6-Me | oil* | 99-100 | 79-80 |
| 144 | Me | 3-MeO-Ph | 6-Me | oil* | 70-1 | 65-6 |
| 145 | Me | 4-Me-Ph | 5-Cl-6-Me | oil* | 156-7 | 79-80 |
| 146 | Me | 2-F-Ph | 6-Me | oil* | 121-2 | 124-5 |
| 147 | Me | 3,5-$Me_2$-Ph | 5-Me | 115-7 | 131-3 | 85-7 |
| 148 | Me | 3,5-$Me_2$-Ph | 6-Me | oil* | 125-6 | 112-3 |
| 149 | Me | 4-F-Ph | 5-Me | 122-3* | 152-4 | 61-2 |
| 150 | Me | 3-F-Ph | 5-Me | 99-100 | 124-5 | oil |
| 151 | Me | 4-F-Ph | 5-Me | 116-8 | 152-4 | 61-2 |
| 152 | Me | Ph | 4-Me | 110-1 | 132-3 | oil |

| Cpd. No. | $R^1$ | $R^2$ | $(R^3)_n$ | m.p.(°) or physical state Compound of formula I | Intermediates A | B |
|---|---|---|---|---|---|---|
| 153 | Me | 4-Me-Ph | 4-Me | 99-100* | 128-30 | 92-3 |
| 154 | Me | 3,5-$Me_2$-Ph | 4-Me | 94-5 | 150-1 | 83-4 |
| 155 | Me | 3,5-$Me_2$-Ph | 4-Me | 82-3* | 150-1 | 83-4 |
| 156 | Me | 2-F-Ph | 6-Me | 113-4+ | 121-2 | 124-5 |
| 157 | Me | 3-MeO-Ph | 4-Me | oil | 95-7 | 101-2 |
| 158 | Me | 3-$PhCH_2O$-Ph | 4-Me | oil | 104-5 | 94-5 |
| 159 | Me | 4-Me-Ph | 5-F | 124-5* | 148-9 | 78-9 |
| 160 | Cl | 3-MeO-Ph | - | 82-3 | 89-92 | 58-60 |
| 161 | Cl | 4-Me-Ph | - | 86-7 | 122-3 | 75-6 |
| 162 | Cl | 4-Me-Ph | - | 122-3* | 122-3 | 75-6 |
| 163 | Cl | 2,5-$Me_2$-Ph | - | 120-2 | 117-8 | 83-4 |
| 164 | Cl | 2,5-$Me_2$-Ph | - | 108-9* | 117-8 | 83-4 |
| 165 | Cl | Ph | - | 100-1 | 266-8[2] | 58-60 |
| 166 | Cl | Ph | - | 127-8* | 266-8[2] | 58-60 |
| 167 | Cl | 2,5-$(CF_3)_2$-Ph | - | 109-10* | 105-7 | 70-2 |
| 168 | Br | 3-MeO-Ph | - | 75-6 | 112-4 | 52-3 |
| 169 | Br | 4-Me-Ph | - | 96-7 | 141-3 | 64-5 |
| 170 | Br | 3,5-$Me_2$-Ph | - | 119-20 | 132-3 | 82-3 |
| 171 | Br | 2,5-$Me_2$-Ph | - | 136-7 | 109-10 | 84-5 |
| 172 | Cl | 2,5-$(CF_3)_2$-Ph | - | 84-5 | 105-7 | 70-2 |
| 173 | Cl | 3-($^nC_6H_{13}OCO$)-Ph | - | 52-4 | oil | oil |
| 174 | Cl | 3-($^nC_6H_{13}OCO$)-Ph | - | oil* | oil | oil |
| 175 | Br | 3-Cl-Ph | - | 121-2 | 135-6 | 123-4 |
| 176 | Br | 3-F-Ph | - | 106-8 | 131-3 | 75-6 |
| 177 | Br | 3-F-Ph | - | 114-6* | 131-5 | 75-6 |
| 178 | Br | 3-Cl-5-Me-Ph | - | 132-3* | | 88-9 |
| 179 | Cl | 4-Et-Ph | - | gum | | oil |
| 180 | Cl | 4-Et-Ph | - | 84-6* | | oil |
| 181 | Cl | 3-MeOCO-Ph | - | oil* | | 86-8 |
| 182 | Cl | 3-MeOCO-Ph | - | oil | | 86-8 |

| Cpd. No. | $R^1$ | $R^2$ | $(R^3)_n$ | m.p.(°) or physical state Compound of formula I | Intermediates A | B |
|---|---|---|---|---|---|---|
| 183 | Br | 3-Cl-5-Me-Ph | - | 110-2 | | 88-9 |
| 184 | Cl | 3-F-5-Me-Ph | - | 92-5 | 129-31 | 74-5 |
| 185 | Cl | 3-F-5-Me-Ph | - | 107-8* | 129-31 | 74-5 |
| 186 | Br | 4-Et-Ph | - | gum | | |
| 187 | Br | 4-Et-Ph | - | gum* | | |
| 188 | Br | 3-MeO-5-Me-Ph | - | 95-7* | | 63-5 |
| 189 | Cl | 3-Cl-5-Me-Ph | - | 95-7 | | 94-5 |
| 190 | Cl | 3-Cl-5-Me-Ph | - | 120-1* | | 94-5 |
| 191 | Br | 3-MeO-5-Me-Ph | - | 62-3 | | 63-5 |
| 192 | Cl | 3-F-Ph | - | 110-2 | | 72-3 |
| 193 | Cl | 3-F-Ph | - | 115-6* | | 72-3 |
| 194 | Cl | 4-Cl-Ph | - | gum | | 83-4.5 |
| 195 | Cl | 3,5-$Me_2$-Ph | - | 121-2.5* | 140-1 | 85.5-6.5 |
| 196 | Cl | 3,5-$Me_2$-Ph | - | 100-2 | 140-1 | 85.5-6.5 |
| 197 | Cl | 3-$^nC_5H_{11}$O-Ph | - | 57-9 | | oil |
| 198 | MeO | 4-Me-Ph | - | 112-3 | 115-6 | 82-3 |
| 199 | MeO | 3-MeO-Ph | - | 88-9 | 124-6 | 77-8 |
| 200 | MeO | 3-MeO-Ph | - | 73-4* | 124-6 | 77-8 |
| 201 | MeO | 3-Cl-Ph | - | 81-2 | 153-4 | 72-3 |
| 202 | MeO | 3-Cl-Ph | - | 84-5* | 153-4 | 72-3 |
| 203 | MeO | 3,5-$Me_2$-Ph | - | 127-8 | 140-1 | 79-80 |
| 204 | MeO | 3,5-$Me_2$-Ph | - | 83-4* | 140-1 | 79-80 |
| 205 | EtO | Ph | - | 101-2 | | 76-78 |
| 206 | MeO | 3-F-Ph | - | 87-8 | 135-6 | 69-70 |
| 207 | MeO | 3-F-5-Me-Ph | - | 100-1 | 135-6 | 82-3 |
| 208 | MeO | 4-Et-Ph | - | 76-7 | | 34-5 |
| 209 | MeO | 4-Et-Ph | - | 60-1* | | 34-5 |
| 210 | MeO | 3-MeOCO-Ph | - | 80-1 | | 95-6 |
| 211 | MeO | 3-Cl-5-Me-Ph | - | 108-9 | 149-51 | 99-101 |
| 212 | MeO | 2,5-$Me_2$-Ph | - | 98-9 | 142-4 | 68-70 |
| 213 | MeO | 2,5-$Me_2$-Ph | - | 120-2* | 142-4 | 68-70 |

| Cpd. No. | $R^1$ | $R^2$ | $(R^3)_n$ | m.p.(°) or physical state Compound of formula I | Intermediates A | B |
|---|---|---|---|---|---|---|
| 214 | EtO | 4-Me-Ph | - | 110-1 | | 91-2 |
| 215 | EtO | 4-Me-Ph | - | 114-5* | | 91-2 |
| 216 | MeO | 3-Me-Ph | - | 81-2 | 130-31 | 65-6 |
| 217 | MeO | 3-Me-Ph | - | 82-3* | 130-1 | 65-6 |
| 218 | MeO | 4-F-Ph | - | 113-4.5 | 113.5-15 | 97-100 |
| 219 | MeO | 3-$PhCH_2O$-Ph | - | gum | 90-91 | 63-4 |
| 220 | MeO | 4-F-Ph | - | 105-7* | 113.5-15 | 97-100 |
| 221 | MeO | 4-$CF_3O$-Ph | - | 68-70 | 118-19.5 | 67-9 |
| 222 | MeO | 4-$CF_3O$-Ph | - | 79-81* | 118-19.5 | 67-9 |
| 223 | MeO | Ph | - | 108-9 | 132-4 | 81.5-3 |
| 224 | MeO | Ph | - | 137-8.5* | 132-4 | 81.5-3 |
| 225 | MeO | 3-$CF_3$-Ph | - | gum | 125-7 | 81-3 |
| 226 | MeO | 3-$CF_3$-Ph | - | 103-4.5* | 125-7 | 81-3 |
| 227 | MeO | 2-$CF_3$-Ph | - | 85-7 | 146-7 | 96-7 |
| 228 | MeO | 2-$CF_3$-Ph | - | 93-5.5* | 146-7 | 96-7 |
| 229 | Me | 4-Me-Ph | 4-Me | 123-4 | 128-30 | 92-3 |
| 230 | MeO | 3-MeO-5-Me-Ph | - | 130-1 | | 85-6 |
| 231 | Me | 3-$PhCH_2O$-Ph | 4-Me | 99-100* | 104-5 | 94-5 |
| 232 | Me | 5-Cl-2-F-Ph | - | 85-7 | 117-20 | 78-80 |
| 233 | Me | 5-Cl-2-F-Ph | - | 85-7* | 117-20 | 78-80 |
| 234 | Me | 3-Br-Ph | - | 97-8* | 136-7 | 104-5 |
| 235 | Me | 2-F-3-$CF_3$-Ph | - | 50-2 | 111-2 | 93-4 |
| 236 | Me | 2-F-3-$CF_3$-Ph | - | 81-2* | 111-2 | 93-4 |
| 237 | Me | 4-MeO-3-Me-Ph | - | 72-4* | | |
| 238 | Me | 3-Br-Ph | - | 115-7 | 136-7 | 104-5 |
| 239 | Me | 3-[3-(4-Cl-Ph)-1,2,4-oxadiazol-5-yl]-Ph | - | 166-8* | 157-9 | 110-20 |
| 240 | $BuSCH_2$ | 3-MeO-Ph | - | oil | | oil |
| 241 | Me | 2-Br-5-F-Ph | - | 96-7* | | |
| 242 | Me | 2-Br-5-F-Ph | - | 97-8 | | |

| Cpd. No. | $R^1$ | $R^2$ | $(R^3)_n$ | m.p.(°) or physical state Compound of formula I | Intermediates A | B |
|---|---|---|---|---|---|---|
| 243 | Me | 2-F-4-$CF_3$-Ph | - | 93-4 | 109-11 | 64-5 |
| 244 | Me | 2-F-4-$CF_3$-Ph | - | 84-5* | 109-11 | 64-5 |
| 245 | Me | 2-F-3-Me-Ph | - | 98-101 | | 70-2 |
| 246 | Me | 2-F-3-Me-Ph | - | 72-5* | | 70-2 |
| 247 | Me | 3-[3-(4-Cl-Ph)-1,2,4-oxadiazol-5-yl]-Ph | - | 123-4 | 157-9 | 110-20 |
| 248 | $MeSCH_2$ | 2,5-$Me_2$-Ph | - | oil | | oil |
| 249 | $MeSCH_2$ | 2,5-$Me_2$-Ph | - | oil* | | oil |
| 250 | Me | 4-Ph-Ph | - | 87-8.5 | 137-8 | 95-6.5 |
| 251 | Me | 4-Ph-Ph | - | 87-8.5* | 137-8 | 95-6.5 |
| 252 | Et | 4-Cl-3-MeO-Ph | - | 108.5-9.5 | 129-30 | 101-2.5 |
| 253 | Me | 4-F-2-$CF_3$-Ph | - | 74-5.5 | 145-7 | 108-9 |
| 254 | Me | 4-F-2-$CF_3$-Ph | - | 70-2* | 145-7 | 108-9 |
| 255 | Me | 2-F-5-MeO-Ph | - | 86-7* | | |
| 256 | Me | 4-Me-Ph | 5-F | 124-5* | 148-9 | 78-9 |
| 257 | Me | 3-(thiazol-2-yl)-Ph | - | gum* | 171-3 | 65-6 |
| 258 | Cl | 2-Me-Ph | - | 121-3 | 120-2 | 73-5 |
| 259 | Cl | 2-Me-Ph | - | 116-7* | 120-2 | 73-5 |
| 260 | Me | 2-(4-Cl-Ph) thiazol-4-yl | - | 85-7 | 175-7 | 73-5 |
| 261 | Cl | 3-$Pr^iO$-Ph | - | oil* | 98-100 | 67-8 |
| 262 | Cl | 5-Cl-2-Me-Ph | - | 112-3* | 142-4 | 120-1 |
| 263 | Me | 2-F-5-MeO-Ph | - | 77-8 | | |
| 264 | Cl | 2-F-5-MeO-Ph | - | 58-9* | 122-3 | 71-3 |
| 265 | Cl | 4-F-Ph | - | 93-4 | 151-2 | 100-1 |
| 266 | Cl | 3-$CF_3$-Ph | - | 58-60 | 118-9 | 81-2 |
| 267 | Me | 4-Me-Ph | 5-F | 102-3 | 148-9 | 78-9 |
| 268 | Me | 4-Me-Ph | 4-F | 115-6 | 134-5 | oil |
| 269 | Me | 4-Me-Ph | 4-F | 97-8* | 134-5 | oil |
| 270 | Me | 3-(thiazol-2-yl)-Ph | - | gum | 171-3 | 65-6 |
| 271 | MeO | 4-Cl-3-MeO-Ph | - | 88-90* | 135-7 | 109-11 |

| Cpd. No. | $R^1$ | $R^2$ | $(R^3)_n$ | m.p.(°) or physical state Compound of formula I | Intermediates A | B |
|---|---|---|---|---|---|---|
| 272 | Cl | 3,5-$Cl_2$-Ph | - | 89-90 | | 111-3 |
| 273 | Cl | 3,5-$Cl_2$-Ph | - | 127-8* | | 111-3 |
| 274 | Cl | 5-Cl-2-Me-Ph | - | 144-6 | 142-4 | 120-1 |
| 275 | MeO | 4-MeO-3-Me-Ph | - | 84-6 | | |
| 276 | Me | 4-Cl-3-$CF_3$-Ph | - | 107.5-9 | 135-6 | 55-6 |
| 277 | Me | 4-Cl-3-$CF_3$-Ph | - | 77-8* | 135-6 | 55-6 |
| 278 | MeO | 3,5-$Cl_2$-Ph | - | 116-8 | 153-4.5 | 120-121.5 |
| 279 | MeO | 3,5-$Cl_2$-Ph | - | 109.5-11* | 153-4.5 | 120-1.5 |
| 280 | MeO | 4-$CF_3$-Ph | - | 83.5-4.5 | 114-5.5 | 85-6 |
| 281 | MeO | 4-$CF_3$-Ph | - | 106.5-8* | 114-5.5 | 85-6 |
| 282 | Me | 3-Ph-Ph | - | 124-6 | 89-91 | 82-3.5 |
| 283 | Me | 3-(Me-C≡C-)-Ph | - | 71.5-85 | 166-8 | 71-83.5 |
| 284 | Me | 4-Cl-3-$CHF_2O$-Ph | - | oil | 114.5-6 | 65.5-6.5 |
| 285 | Me | 5-Cl-2-Me-Ph | - | 137-8 | 116-7 | 90-1 |
| 286 | Me | 5-Cl-2-Me-Ph | - | 121-2* | 116-7 | 90-1 |
| 287 | Me | 2-Cl-5-MeO-Ph | - | 91-2 | 141-2 | 83-4 |
| 288 | Cl | 2-F-5-MeO-Ph | - | oil | 122-3 | 71-3 |
| 289 | Cl | 2-Cl-5-MeO-Ph | - | 97-8 | 143-5 | 78-91 |
| 290 | Cl | 2-Cl-5-MeO-Ph | - | 80-1* | 143-5 | 79-81 |
| 291 | MeO | 4-Cl-3-MeO-Ph | - | 116-8 | 135-7 | 109-11 |
| 292 | Me | 3-$Bu^t$-5-Me-Ph | - | oil | oil | oil |
| 293 | Me | 3-$Bu^t$-5-Me-Ph | - | oil* | oil | oil |
| 294 | Me | 3-(Me-C≡C)-Ph | - | 120.5-4* | 166-8 | 71-83.5 |
| 295 | Me | 3-cyclopropyl-Ph | - | gum | 115.5-8 | oil |
| 296 | Me | 5-Cl-2-MeO-Ph | - | 100-1 | 129-30 | 99-100 |
| 297 | Me | 5-Cl-2-MeO-Ph | - | 92-3* | 129-30 | 99-100 |
| 298 | Me | 2-Br-5-Me-Ph | - | 137-8 | 121-2 | 63-4 |
| 299 | Me | 2-Br-5-Me-Ph | - | 124-5* | 121-2 | 63-4 |
| 300 | Me | 3-(2-thiazolin-2-yl)-Ph | - | 110-1 | | 72-4 |
| 301 | Cl | 2,5-$Cl_2$-Ph | - | 142-3 | 115-20 | 112-4 |
| 302 | Cl | 2,5-$Cl_2$-Ph | - | 103-4* | 115-20 | 112-4 |

| Cpd. No. | $R^1$ | $R^2$ | $(R^3)_n$ | m.p.(°) or physical state Compound of formula I | Intermediates A | B |
|---|---|---|---|---|---|---|
| 303 | Et | 4-Cl-3-MeO-Ph | - | gum* | 129-30 | 101-2.5 |
| 304 | Me | 3-Ph-Ph | - | oil* | 89-91 | 82-3.5 |
| 305 | MeO | 2,5-$Cl_2$-Ph | - | 119-20* | 159-60 | 104-5 |
| 306 | MeO | 2,5-$Cl_2$-Ph | - | 94-5.5 | 159-60 | 104-5 |
| 307 | Me | 3-cyclopropyl-Ph | - | 56.5-8.5* | 115.5-8 | oil |
| 308 | Cl | 3-Me-Ph | - | 88-90* | 133-4 | 80-1 |
| 309 | Cl | 4-$CF_3$-Ph | - | 85-6 | 135-7 | 62-3 |
| 310 | Cl | 4-$CF_3$-Ph | - | oil* | 135-7 | 62-3 |
| 311 | Me | 2,5-$Br_2$-Ph | - | 124-5 | 90-5 | 107-9 |
| 312 | Me | 2,5-$Br_2$-Ph | - | 87-8* | 90-5 | 107-9 |
| 313 | Me | 3-[PhO-$(CH_2)_2$-O]-Ph | - | 95-7 | | |
| 314 | Cl | 4-Me-Ph | 5-Me | 128-9 | | 85-6 |
| 315 | Me | 4-[MeO-$(CH_2)_2$-O]-Ph | - | 96-7 | | oil |
| 316 | Me | 3-(CH≡C-$CH_2$)-Ph | - | 83-5 | | |
| 317 | Me | 2-MeS-5-Cl-Ph | - | 92-4 | 143-5 | 89-90 |
| 318 | Me | 2-MeS-5-Cl-Ph | - | 93.5-4.5* | 143-5 | 89-90 |
| 319 | Me | 4-MeS-Ph | - | 93-4 | 128-30 | 81-3 |
| 320 | Me | 4-MeS-Ph | - | 104-6* | 128-30 | 81-3 |
| 321 | Me | 4-F-Ph | - | 124-5 | 117-9 | 78-80 |
| 322 | Me | 4-F-Ph | - | 118-20* | 117-9 | 78-80 |

² = Na salt

+ = contains 14% Z-isomer

## Example 2

A mixture of 7-methylbenzo[b]thiophen-2(3H)-one (5.0 g), sodium hydroxide (2.44 g) and water (50 ml) was heated under reflux for one hour. 4-Methylbenzyl chloride (4.49 g) was added and the mixture refluxed for a further 1½ hours. It was then cooled, acidified with hydrochloric acid and the solid precipitate filtered off, washed with water and dried to give [2-(4-methylbenzylthio)-3-methyl-phenyl]acetic acid, m.p. 110-2°.

To a solution of this compound (7.1 g) in dichloromethane (50 ml) was added triethylamine (2.62 g), followed by methyl chloroformate (2.43 g). The mixture was heated under reflux for 0.5 hours, cooled, washed with water and aqueous sodium hydrogen carbonate, dried and evaporated to give methyl [2-(4-methylbenzylthio)-3-methylphenyl]-acetate, as an oil.

This compound (5.5 g) was added to a stirred suspension of sodium hydride (0.58 g, 80% dispersion in mineral oil) in dimethylformamide (75 ml). After a further 1 hour methyl formate (5.5 g) was added dropwise. The mixture was stirred overnight at room temperature to give methyl [(E)-3-hydroxy-2-[2-(4-methylbenzylthio)-3-methylphenyl]prop-2-enoate, which was not isolated. Methyl iodide (2.73 g) was added, the reaction mixture stirred for 3 hours and evaporated. Diethyl ether and water were added, the organic phase

separated and washed with water, dried and evaporated to give methyl [(E)-3-methoxy-2-[2-(4-methylbenzylthio)-3-methyl-phenyl]prop-2-enoate, m.p. 109-11°, (compound 2a).

Example 3

In a similar manner to Example 1, there was obtained 2-[2-(4-methylbenzylthio)-3-methylphenyl]-2-oxoacetic acid, m.p. 128-9°, which was converted to the methyl ester, m.p. 42-3°. Methoxyamine hydrochloride (1.67 g) was added to a solution of this (6 g) in methanol (100 ml) and the mixture heated under reflux, with stirring, for 24 hours. The mixture was poured into water and extracted with ether. The extract was dried and evaporated and the residue recrystallised from diisopropyl ether up to give methyl 2-methoxyimino-2-[2-(4-methylbenzylthio)-3-methyl-phenyl]acetate, m.p. 93-5°. (Compound 400)

In a similar manner there was obtained:

(i) methyl 2-[3-chloro-2-(4-methylbenzyl-thio)phenyl]-2-(methoxyimino)acetate, m.p. 86-7°. (Compound 401) and

(ii) methyl 2-[3-bromo-2-(4-methylbenzylthio)phenyl]-2-(methoxyimino)-acetate, m.p. 97-8°. (Compound 402)

Example 4

In a similar manner to Example 1, 7-methylbenzo[b]-thiophene-2,3-dione was reacted with 1-bromo-6,6-dimethyl-hept-2-en-4-yne to give 2-[2-(6,6-dimethylhept-2-en-4-ynylthio)-3-methylphenyl]-2-oxoacetic acid, as an oil. This was obtained as a 2:1 mixture of the E:Z isomers. This was then methylated to give the methyl ester, as an oil, in a 2:1 mixture of the E:Z isomers. This in turn was converted to methyl 2-[2-(6,6-dimethylhept-2-en-4-ynyl-thio)-3-methylphenyl]-3-methoxyprop-2-enoate, as an oil. The oil was subjected to chromatographic separation and the following isomers were obtained:

i) E-propenoate-E/Z-enyne (compound 451a)
ii) Z-propenoate-E-enyne, (compound 451b) and
iii) Z-propenoate-Z-enyne (compound 451c).

In a similar manner to Example 1, 7-chlorobenzo[b]-thiophene-2,3-dione was reacted with 1-bromo-6,6-dimethyl-hept-2-en-4-yne to give 2-[3-chloro-2-(6,6-dimethylhept-2-en-4-ynylthio)phenyl]-2-oxoacetic acid, as an oil. This was obtained as a 2:1 mixture of the E:Z isomers. This was then methylated to give the methyl ester, as an oil, in a 2:1 mixture of the E:Z isomers. This in turn was converted to methyl 2-[3-chloro-2-(6,6-dimethyl-hept-2-en-4-ynyl-thio)phenyl]-3-methoxyprop-2-enoate, as an oil. The oil was subjected to chromatographic separation and the following isomers were obtained:

i) E-propenoate-E-enyne (compound 452a)
ii) E-propenoate-Z-enyne, (compound 452b) and
iii) a 2:1 mixture of Z-propenoate-E-enyne and Z-propenoate-Z-enyne (compound 453c).

In a similar manner, there was obtained:

2-[3-bromo-2-(6,6-dimethylhept-2-en-4-ynylthio)phenyl]-2-oxoacetic acid, as an oil, obtained as a 2:1 mixture of the E:Z isomers, which was then methylated to give the methyl ester, as an oil, in a 2:1 mixture of the E:Z isomers. This in turn was converted to methyl 2-[3-bromo-2-(6,6-dimethyl-hept-2-en-4-ynylthio)phenyl]-3-methoxy-prop-2-enoate, as an oil. The oil was subjected to chromatographic separation and the following isomers were obtained, as oils:

i) E-propenoate-E/Z-enyne (compound 453a),
ii) E-propenoate-Z-enyne, (compound 453b), and
ii) E-propenoate-Z-enyne, (compound 453c).

Example 5

In a similar manner to that described in Example 1, 7-methylbenzo[b]thiophen-2(3H)-one was reacted with 2-bromomethyl-5-phenylpyrimidine to give crude [2-(5-phenylpyrimidin-2-ylmethylthio)-3-methylphenyl]acetic acid, which was esterified to give the methyl ester. A mixture of this product (5 g), dimethylformamide dimethylacetal (2.5 ml) and pyridinium tosylate (0.4 g) in toluene (100 ml) was heated at 105° for 2½ hours. The mixture was distilled to remove methanol, toluene and then the excess dimethylformamide dimethylacetal was evaporated in vacuo. Further dimethylformamide dimethylacetal (1.8 ml) was added and the mixture heated at 105° for a further 2 hours. The mixture was again distilled and the cycle repeated three times. The mixture was evaporated and the residue worked up to give methyl 3-dimethylamino-2-[2-(5-phenylpyrimidin-2-ylmethylthio)-3-methylphenyl]prop-2-enoate. A mixture of this

crude product (1 g), dissolved in acetone (100 ml) and Amberlyst 15 (an acidic ion-exchange resin; 5 g) and water (0.5 ml) was stirred at room temperature for 1 hour. The mixture was filtered and the filtrate evaporated to give crude methyl 3-hydroxy-2-[2-(5-phenylpyrimidin-2-ylmethyl-thio)-3-methylphenyl]prop-2-enoate. This (0.4 g) was dissolved in dimethylformamide (20 ml) and sodium hydride (0.44 g of 60% dispersion in oil) was added and the mixture stirred for 1 hour at room temperature. A solution of methyl iodide (1 ml) in dimethylformamide (19 ml) was added and the mixture stirred at room temperature overnight. Solvent was evaporated and the resulting oil was partitioned between ether and water and the organic phase washed with water, dried and evaporated. The residue was purified by silica gel chromatography to give methyl 3-methoxy-2-[2-(5-phenylpyrimidin-2-ylmethylthio)-3-methylphenyl]prop-2-enoate, m.p. 124-7°. (compound 5a)

In a similar manner there was obtained:

a) methyl 3-methoxy-2-{2-[5-(4-chlorophenyl)pyrimidin-2-ylmethylthio]-3-methylphenyl}prop-2-enoate, m.p. 123-5°. (compound 5b), and

b) methyl 3-methoxy-2-[2-(3-methyl-5-nitrobenzylthio)-3-methylphenyl]prop-2-enoate, m.p. 135-6°. (compound 5c)

Example 6

This example illustrates an alternative method of preparing compound 163.

A solution of sodium methoxide (10.1.g) in ethanol (300 ml) was added to a mixture of intermediate A to compound 163 (6.2.g) and hydrazine hydrate (4.65 g) with stirring. The mixture was heated, ethanol and excess hydrazine hydrate distilled off and the residue maintained at 185° for 15 minutes. Excess 5% hydrochloric acid was added to the cooled residue, followed by diethyl ether. The organic phase was separated, washed with water, dried over magnesium sulfate, evaporated and the residue recrystallised from diisopropyl ether to give [3-chloro-2-(2,5-dimethylbenzylthio)phenyl]acetic acid, m.p. 132-3°.

Then, following the method described in Example 2, this was converted to the methyl ester, m.p. 79-80°, which in turn was converted to methyl [(E)-3-hydroxy-2-[3-chloro-2-(2,5-dimethylbenzylthio)phenyl]-prop-2-enoate,
m.p. 76-8°, which then was methylated to give compound 163.

Example 7

Meta-chloroperbenzoic acid (0.128 g, 60%) in dichloromethane (5 ml) was added dropwise to a stirred solution of compound 248 (0.18 g) in dichloromethane (10 ml). The resultant solution was then stirred for 30 minutes at room temperature. The reaction mixture was washed with 10% aqueous sodium bicarbonate, the organic layer dried over magnesium sulfate and the solvent removed in vacuo to give a yellow oil. The product was purified by silica gel flash chromatography with ethyl acetate as the eluent to give methyl 2-[2-(2,5-dimethylbenzylthio)-3-(methylsulfinylmethyl)phenyl]-3-methoxyprop-2-enoate, as a yellow oil. (compound 7a)

In a similar manner, there was obtained from compound 240, methyl 2-[3-(butylsulfinylmethyl)-2-[(3-methoxybenzyl)-thio]phenyl]-3-methoxyprop-2-enoate, m.p. 94-7°. (compound 7b)

Preparation of starting materials

7-Methylbenzo[b]thiophen-2(3H)-one

7-Methylbenzo[b]thiophene (18.95 g) was dissolved in ether under nitrogen and the solution cooled to 0°. Butyl lithium (51.1 ml of 2.5M in hexane) was added whilst maintaining the temperature at 0-5°. The mixture was stirred at 0° for 10 minutes, then for 45 minutes at room temperature. It was cooled to 0° and tributyl borate (34.3 ml) added dropwise. After stirring for 1 hour at 0°, the mixture was warmed to room temperature and allowed to stand overnight. It was cooled and 2M hydrochloric acid (225 ml) added. The aqueous phase was extracted with ether and the extract extracted with aqueous sodium hydroxide (2M). The basic extract was acidified with concentrated hydrochloric acid to pH 2. The precipitate was filtered and washed with water to give tris-(7-methylbenzo[b]-thiophen-2-yl)cyclotriboroxane. This product (21.4 g) was suspended in water, the mixture heated to 50° and hydrogen peroxide (19.7 ml) added dropwise whilst maintaining the temperature between 55° and 60°. The temperature was kept at 60-68° after the addition. After 1 hour, the mixture was allowed to cool down to room temperature, then extracted with ethyl acetate. The extract was washed with aqueous sodium sulfite (10%), dried and evaporated. The residue was purified

by silica gel column chromatography (ethyl acetate:hexane 1:2), followed by recrystallisation from hexane to give 7-methylbenzo[b]thiophen-2(3H)-one.

7-Ethylbenzo[b]thiophene-2,3-dione

2-Ethylbenzenethiol (9.10 g) was added dropwise with stirring and cooling to a solution of oxalyl chloride (12.56 g) in diethyl ether (100 ml). The mixture was stirred at room temperature for 1 hour then evaporated.

The residual oil was dissolved in carbon disulfide (100 ml) and cooled to 5°C. Aluminium chloride (13.2 g) was added portionwise over 10 minutes with stirring. The mixture was allowed to warm to room temperature. A black tar was deposited and the stirring became difficult. The mixture was warmed on a warm water bath for 20 minutes, cooled and the carbon disulfide decanted off. Hydrochloric acid (1M) was added and the black tar decomposed to give a sticky red solid. This was extracted with ethyl acetate and the extract washed with water, dried and evaporated to give 7-ethylbenzo[b]thiophene-2,3-dione, m.p. 57-58°.

In a similar manner, there was obtained:
5,7-dimethylbenzo[b]thiophene-2,3-dione, m.p. 148-50°.
and 4,7-dimethylbenzo[b]thiophene-2,3-dione. m.p. 123-4°.

5-Chloro-4,7-dimethylbenzo[b]thiophene-2,3-dione

4-Chloro-2,5-dimethylbenzenethiol (40.9 g) was added to 30% sodium hydroxide solution (200 ml). The mixture was stirred vigorously and chloroacetic acid (45.3 g) was added portionwise. The mixture was stirred at 90° for 2 hours, cooled and poured onto ice (750 g). Concentrated hydrochloric acid (60 ml) was added with brisk stirring. The white solid was filtered, washed well with water and dried in vacuo to give (4-chloro-2,5-dimethylphenyl-thio)acetic acid. This was suspended in chlorobenzene (300 ml) and phosphorus trichloride (41.2 g) was added dropwise with stirring. The mixture was heated on a steam bath for 40 minutes and the supernatant liquid decanted from a sticky yellow oil. The supernatant was cooled to room temperature and aluminium trichloride (33.8 g) was added portionwise. The mixture was stirred and heated on a steam bath for 30 minutes to give a deep red solution, poured onto ice (1000 g) and filtered. The solid was washed with water to give 5-chloro-4,7-dimethylbenzo[b]-thiophen-3(2H)-one. This was suspended in 10% sodium hydroxide solution (400 ml) and warmed to 60°. A suspension of N,N-dimethyl-4-nitrosoaniline (34.3 g) in 5% hydrochloric acid (200 ml) was added with vigorous stirring. The mixture was stirred for 30 minutes and filtered. The purple solid was washed with water, treated with 15% hydrochloric acid (700 ml) and the mixture heated under reflux with stirring for 45 minutes, cooled, filtered and the solid washed with water. It was then treated with 20% aqueous sodium carbonate (700 ml) and the mixture heated under reflux with stirring for 40 minutes. It was filtered hot to remove purple solid by-product. The cooled filtrate was acidified to give an orange solid which was filtered and washed well with water and dried in vacuo to give the title product, m.p. 144-5°.

7-methoxybenzo[b]thiophene-2,3-dione

A solution of 2-methoxybenzenethiol (21 g) in ether (100 ml) was added dropwise to a mixture of N,N,N',N'-tetramethylethylenediamine (34.9 g) and butyl lithium (2.5 M in hexane) (120 ml) in ether (500 ml) at 0° under nitrogen. The mixture was stirred at room temperature for 18 hours and then cooled to -40°. A solution of dimethyl oxalate (17.7 g) in ether (200 ml) was added dropwise. The reaction mixture was stirred at room temperature for 1 hour, poured onto crushed ice and stirred vigorously. The aqueous layer was acidified to pH 1, extracted with dichloromethane and the extract dried and evaporated. The residue was purified silica gel column chromatography to give the title compound, m.p. 156-157°.

In a similar manner there was obtained 7-ethoxy-benzo[b]thiophene-2,3-dione, m.p. 116-8°.

7-chlorobenzo[b]thiorhene-2,3-dione

Sodium nitrite (203 g) was added portionwise with stirring at room temperature over 2 hours to a solution of 7-chlorobenzo[b]thiophen-3(2H)-one (113g) in acetic acid (1200 ml). The mixture was stirred for 2 hours, poured into ice/water (2000 ml), the solid filtered off, washed well with water and filtered dry to give 7-chlorobenzo[b]thiophene-2,3-dione 2-oxime, m.p. 205-10°. This was then treated with 50% sulfuric acid at reflux, with stirring for 2 hours. The mixture was cooled and poured into ice/water (2500 ml), filtered and

washed well with water and recrystallised from ethanol to give the title product.

In a similar manner there was obtained:

a) 5-fluoro-7-methylbenzo[b]thiophene-2,3-dione 2-oxime, m.p. 220° (dec), which was converted to the dione, m.p. 105-6°,

b) 6-fluoro-7-methylbenzo[b]thiophene-2,3-dione 2-oxime, m.p. 218-20° (dec), which was converted to the dione, m.p. 101-2°, and

c) 7-chloro-5-methylbenzo[b]thiophene-2,3-dione 2-oxime, m.p. 230° (dec), which was converted to the dione, m.p. 152-3°,

## Example 8

Tests were carried out to determine the photostability of a number of compounds of the inventions. Acetone solutions of the compounds were applied to pre-coated silica-gel tlc plates at a rate of 10 $\mu g/cm^{-2}$. After the solvent had evaporated the plates were exposed for one hour in a Sol 2 sunlight simulator (manufactured by Dr K Hönle GmbH, Germany). The plates were then developed in dichloromethane/ethyl acetate (95:5). For the purpose of comparison, certain analogues were similarly treated in which $R^1$ is hydrogen and in some cases the phenyl is substituted by methyl at the 4 position.

Examination of the plates of the comparison compounds indicated formation of significant amounts of sulfoxide, whereas in the case of the compounds of the invention, little or no sulfoxide was obtained.

The compounds tested were as follows:

| a) $R^2$ = Ph | | |
|---|---|---|
| Compound No | $R^1$ | $(R^3)_n$ |
| 11 | Me | - |
| 19 | Me | - |
| 69 | Et | - |
| 152 | Me | 4-Me |
| 205 | EtO | - |
| Comparison | H | - |

| b) $R^2$ = 3-MeO-Ph | | |
|---|---|---|
| Compound No | $R^1$ | $(R^3)_n$ |
| 4 | Me | - |
| 34 | Et | - |
| 139 | Me | 5-Me |
| 144 | Me | 6-Me |
| 157 | Me | 4-Me |
| 160 | Cl | - |
| 168 | Br | - |
| 199 | MeO | - |
| Comparison | H | - |
| | H | 4-Me |

| c) $R^2$ = 4-Me-Ph | | |
|---|---|---|
| Compound No | $R^1$ | $(R^3)_n$ |
| 2 | Me | - |
| 16 | Et | - |
| 138 | Me | 5-Me |
| 161 | Cl | - |
| 169 | Br | - |
| 198 | MeO | - |
| 214 | EtO | - |
| 229 | Me | 4-Me |
| Comparison | H | - |
| | H | 4-Me |

| d) $R^2$ = 3-$CF_3$-Ph | | |
|---|---|---|
| Compound No | $R^1$ | $(R^3)_n$ |
| 55 | Me | - |
| 225 | MeO | - |
| 226 | MeO | - |
| Comparison | H | - |

| e) $R^2$ = 3-F-Ph | | |
|---|---|---|
| Compound No | $R^1$ | $(R^3)_n$ |
| 5 | Me | - |
| 32 | Et | - |
| 108 | Me | - |
| 150 | Me | 5-Me |
| 176 | Br | - |
| 192 | Cl | - |
| 206 | MeO | - |
| Comparison | H | - |

Test Example A

Compounds are assessed for activity against one or more of the following:

Phytophthora infestans: late tomato blight (PI)
Plasmopara viticola: vine downy mildew (PV)
Erysiphe graminis: barley powdery mildew (EG)
Pyricularia oryzae: rice blast (PO)
Pellicularia sasakii: rice sheath blight (PS)
Botrytis cinerea: grey mould of tomato (BC)
Venturia inaequalis: apple scab (VI)
Leptosphaeria nodorum: glume blotch (LN)
Pseudocercosporella herpotrochoides; eyespot (PH)

Aqueous solutions or dispersions of the compounds at the desired concentration, including a wetting agent, were applied by spray or by drenching the stem base of the test plants. These plants were then inoculated with appropriate test pathogens and kept under controlled environment conditions suitable for maintaining plant growth and development of the disease. After an appropriate time, the degree of infection of the leaf surface or stem base, as appropriate, was visually estimated. Compounds were considered active if they gave greater than 50% control of the disease at a concentration of 500 ppm (w/v) or less.

Activities were demonstrated as follows (+ = active).

| Compound | PI | PV | EG | PO | PS | BC | VI | LN | PH |
|---|---|---|---|---|---|---|---|---|---|
| 1 | + | + | + | + | | + | + | + | + |
| 2 | | + | | | | | + | | |
| 3 | | + | + | + | | | + | | |
| 4 | + | + | + | + | | | + | | |
| 5 | + | + | | + | | | + | + | + |
| 6 | + | + | | | | | + | | |
| 7 | + | + | | + | | | + | | |
| 8 | | | + | | + | | + | | |
| 9 | + | + | | + | + | | + | | + |
| 10 | + | + | + | + | + | | + | | |
| 11 | + | + | + | | | + | + | | |
| 12 | | | | | | | + | | |
| 14 | | + | + | + | | | + | + | |
| 15 | | + | | | | | + | | |
| 16 | | + | + | | | + | + | | + |
| 17 | | + | + | | + | | + | + | |
| 18 | | + | | | | | + | | |
| 19 | | + | | | | | + | + | |
| 20 | + | + | | + | | | + | | |
| 21 | + | + | | | | | + | + | |
| 22 | | | | | | | + | + | |
| 24 | | + | | | | | + | | |
| 25 | | + | + | | | | + | + | + |
| 27 | | + | + | | | | + | + | + |
| 28 | | + | | | | | + | | |
| 30 | | + | | | | + | + | + | |
| 32 | | + | | | | | + | + | + |
| 33 | | + | | + | | | + | | |
| 34 | | | | | | | + | | |
| 35 | | | | | | | + | | |
| 37 | | | + | | | | + | | |

| Compound | PI | PV | EG | PO | PS | BC | VI | LN | PH |
|---|---|---|---|---|---|---|---|---|---|
| 38 | + | | + | | | | + | | + |
| 39 | + | + | | | | | + | | |
| 41 | | | + | | | + | + | | |
| 42 | | | | | | | + | | |
| 43 | | | | | | + | | | |
| 45 | | + | + | | | + | | | |
| 47 | + | + | + | | | + | + | | |
| 49 | + | + | + | | + | + | + | | |
| 51 | | | + | | | + | + | | + |
| 53 | | + | | | | | | | |
| 54 | + | + | + | | | + | + | | |
| 55 | | + | + | | | | + | | |
| 56 | | + | + | | | + | + | + | |
| 57 | | + | + | | | | | | |
| 58 | | + | + | | | | + | | |
| 59 | | + | + | | | + | + | | |
| 60 | + | + | + | | | + | + | | |
| 61 | | + | + | | | | | | |
| 62 | | | + | | | | + | | + |
| 63 | | + | + | | | | + | | + |
| 64 | | + | + | | | | + | | |
| 65 | + | + | | | | | | | |
| 66 | + | + | | | + | | + | | |
| 67 | | + | + | | | + | + | | |
| 69 | | + | | | | + | + | | |
| 70 | | + | | | | | | | |
| 71 | | | | + | | + | + | | |
| 72 | | + | | | + | + | + | | |
| 73 | | + | + | | | | | + | |
| 74 | | + | | | | | + | | |
| 75 | | + | | | | | + | | |
| 76 | | + | + | | | | + | | |

| Compound | PI | PV | EG | PO | PS | BC | VI | LN | PH |
|---|---|---|---|---|---|---|---|---|---|
| 77 | | | | | | + | | | |
| 78 | | + | | + | | | + | | + |
| 79 | | | | + | | | + | | |
| 80 | | | | | | | + | | |
| 81 | | + | + | + | | + | + | | |
| 82 | | | | + | | | + | | |
| 83 | + | + | + | | | | + | | + |
| 84 | | + | | | | | + | | |
| 85 | | | | | | + | + | + | |
| 86 | | | | | | + | + | + | |
| 87 | | | + | | | | | | |
| 88 | | + | + | | | | + | | + |
| 89 | | + | + | | | | + | + | + |
| 90 | | + | + | | + | + | + | | + |
| 91 | | + | + | | | | + | | |
| 92 | | + | + | | | | + | | |
| 93 | + | + | + | | | + | + | | + |
| 94 | | | + | | | | | | |
| 95 | + | + | + | + | | | + | | + |
| 97 | | + | + | | | | + | | |
| 100 | | + | + | | | | + | | |
| 101 | | + | + | | | | + | | |
| 102 | | + | + | | | | | | |
| 103 | | + | + | | | | | | |
| 104 | | + | + | | + | | + | | |
| 105 | | + | + | | + | + | + | | |
| 136 | | + | | | | | | | |
| 137 | | | + | + | | + | + | | |
| 138 | | + | | | | | + | | |
| 139 | | + | | | | | + | + | |
| 140 | | + | | | | | + | + | |
| 141 | | + | | | + | | + | | |

| Compound | PI | PV | EG | PO | PS | BC | VI | LN | PH |
|---|---|---|---|---|---|---|---|---|---|
| 142 | | + | | | | | + | | |
| 143 | | + | | | | | + | + | |
| 145 | | + | | | | | + | | |
| 146 | + | + | | | | | | + | |
| 147 | | + | | + | | | + | + | |
| 148 | | | | | | | + | | |
| 149 | | | | | | + | | | |
| 150 | + | + | | | | | + | | |
| 151 | + | | | | | | + | | |
| 152 | | + | | | | | + | | |
| 154 | | + | + | | | | + | | |
| 155 | | | | | | + | | | |
| 156 | | + | + | | | | | | |
| 160 | | + | | | | + | + | | |
| 161 | | + | + | + | + | + | + | + | + |
| 163 | | + | + | | | | + | | |
| 165 | | | + | + | | + | + | + | |
| 167 | | + | + | | | | + | | |
| 168 | + | + | + | | | | + | + | |
| 169 | | + | + | | | + | + | + | |
| 170 | | + | + | | | | + | + | |
| 171 | | | + | | | + | + | + | |
| 172 | | | + | | | | + | + | |
| 173 | | + | | | | | + | | |
| 174 | | + | | | | | + | | |
| 175 | | | + | + | | | + | | |
| 176 | + | + | | | | | + | + | |
| 177 | + | + | | | | | | | |
| 178 | | | | | | | | + | |
| 179 | | + | + | | | | + | | |
| 180 | | | | | | + | + | | |
| 181 | + | + | | | | | | | |

| Compound | PI | PV | EG | PO | PS | BC | VI | LN | PH |
|---|---|---|---|---|---|---|---|---|---|
| 182 | | + | | | | | | | |
| 183 | + | + | | | | | | | |
| 184 | | + | + | | | | + | | |
| 185 | | | + | | | | | | |
| 186 | + | + | | | | | + | + | + |
| 187 | | + | | | | + | + | | |
| 188 | | | | | | | + | | |
| 189 | | + | + | | | + | + | + | |
| 190 | | + | | | | | | | |
| 191 | | + | | | | | + | | |
| 192 | | + | | | | | + | | |
| 193 | | | | | | | + | | |
| 195 | + | | | | | | + | | + |
| 197 | | + | | | | | + | | |
| 198 | | + | + | | | + | + | + | |
| 199 | | + | | | | + | + | + | + |
| 200 | | + | | | | + | + | | |
| 201 | | + | | + | | | + | | |
| 202 | | + | + | + | | | + | | |
| 203 | | | + | | | | + | | |
| 204 | | + | + | | | | + | | |
| 205 | | + | | | | | + | | |
| 206 | | | + | | | | + | | |
| 207 | | + | + | | | + | + | | |
| 208 | | + | | | | | + | | |
| 210 | + | + | + | | | | + | | + |
| 211 | + | + | + | | | | + | | + |
| 212 | | + | + | | | + | + | + | + |
| 213 | | | | | | | + | | |
| 214 | | | | | | + | | | |
| 216 | | + | | | | | + | | |
| 217 | | | | | | | + | | |

| Compound | PI | PV | EG | PO | PS | BC | VI | LN | PH |
|---|---|---|---|---|---|---|---|---|---|
| 218 | + | + | + | | | + | + | | |
| 219 | + | + | | | | | | | |
| 220 | | + | | | | + | + | | |
| 221 | | + | | | | | + | | |
| 222 | | + | | | | | | | |
| 223 | | + | + | | | | + | + | |
| 225 | | + | + | | | | + | + | |
| 226 | | + | + | | | | | | |
| 227 | + | + | + | | | | + | + | |
| 228 | | + | + | | | | | + | |
| 229 | | + | | + | | + | + | | |
| 230 | | + | | | | + | + | + | |
| 231 | | + | | | | | | + | |
| 232 | + | + | + | + | | | + | + | |
| 233 | + | | + | | | | + | | |
| 234 | | + | + | | | | | | |
| 235 | + | + | + | | | | + | | |
| 236 | | + | | | | | | | |
| 237 | | | + | | | | + | | |
| 238 | + | | + | | | | + | | |
| 239 | | | + | | | | | | |
| 240 | | + | + | | | | + | | |
| 241 | | + | + | | | | | | |
| 242 | + | | + | | | | + | | |
| 243 | + | | + | | | | + | + | |
| 244 | | | | | | | + | | |
| 245 | | + | | | | | + | | |
| 247 | | + | + | | | | | | |
| 248 | | | + | | | | | | |
| 250 | + | + | + | | | + | | | |
| 251 | | + | | | | | | + | |
| 252 | + | + | + | | | + | | | |

| Compound | PI | PV | EG | PO | PS | BC | VI | LN | PH |
|---|---|---|---|---|---|---|---|---|---|
| 253 | + | + | + | | | + | | | |
| 254 | + | + | + | | | + | | | |
| 255 | | | + | | | | | | |
| 257 | | | | | | | + | | |
| 258 | | + | + | | | | + | | |
| 259 | | + | | | | | | | |
| 260 | | | + | | | | | | |
| 263 | + | + | + | | | | + | | |
| 264 | | + | | | | | | | |
| 265 | | + | | | | | + | | |
| 266 | | + | | | | | + | | |
| 267 | | + | | | | | | | |
| 268 | | + | | | | | + | | |
| 270 | | | | | | | + | | |
| 271 | | + | | | | | + | | |
| 272 | | + | | | | | + | | |
| 274 | | + | | | | | + | | |
| 275 | + | + | + | | | | + | | |
| 276 | | + | | | | | | | |
| 277 | | + | | | | | | | |
| 278 | | + | | | | | + | | |
| 279 | | + | | | | | + | | |
| 280 | | + | | | | | + | | |
| 281 | | + | | | | | + | | |
| 282 | | + | | | | | + | | |
| 313 | | + | + | | | | | | |
| 314 | | + | | | | | | | |
| 400 | | + | + | | | + | + | | |
| 401 | | + | | + | | + | + | + | |
| 402 | | + | + | + | | | + | + | |
| 451a | | | + | | | | | | |
| 451c | | | | | | + | | | |

| Compound | PI | PV | EG | PO | PS | BC | VI | LN | PH |
|---|---|---|---|---|---|---|---|---|---|
| 452a | | + | | | | | + | | |
| 452b | | | | | | | + | | |
| 452c | | | | + | | | | | |
| 453a | + | | | + | | | | | |
| 2a | + | + | | | + | + | + | | |
| 5a | | + | | | | + | + | + | |
| 5b | | + | + | | | + | + | + | + |
| 5c | | + | | | | | + | | |

The following intermediates demonstrated activity as follows:

| Intermediate to Compound No (A or B type) | PI | PV | EG | PO | PS | BC | VI | LN | PH |
|---|---|---|---|---|---|---|---|---|---|
| 1B | | | | | | | + | | |
| 2A | | | | + | | | + | + | |
| 3B | | + | | + | | + | + | | |
| 4B | | + | | + | | | | | |
| 5A | | | | | | | + | | |
| 5B | | + | + | | | | + | + | |
| 6A | | | | + | | | | + | |
| 6B | | | | + | | | | + | + |
| 8B | | | | + | | | | | |
| 9A | | | | | | | + | | |
| 10B | | | + | | | | | | |
| 14A | | | | | | | | + | |
| 14B | | | | | | | + | | |
| 17A | | + | | | | | | | |
| 20A | | + | | | | | | | |
| 20B | | + | | | | | + | | |
| 22B | | | | | | | + | | |
| 24B | | + | | | | | | | |
| 29B | | | | + | | | | + | |
| 32A | | | | | | | + | + | |
| 32B | | + | | | | | | | |
| 38A | | + | | | | | | | |
| 38B | | + | | | | | + | | |
| 42A | | + | + | + | | | | | |
| 42B | | + | + | + | | | + | | |
| 45A | | + | | | | + | | | |
| 45B | | | | | | | + | | |
| 49B | | | | | | | + | | |

| Intermediate to Compound No (A or B type) | PI | PV | EG | PO | PS | BC | VI | LN | PH |
|---|---|---|---|---|---|---|---|---|---|
| 47A | | + | | | | | | | |
| 51B | | | | | + | + | | | |
| 52A | | + | | | | | + | | |
| 52B | | | | | | + | | | |
| 53A | | + | | + | | | + | | |
| 54A | + | + | | | | | | | |
| 55A | | + | | | | | + | | |
| 55B | | + | | | | | + | | |
| 56A | | + | | | | | | | |
| 56B | | + | | | | | | | |
| 58A | | | + | | | | | | |
| 58B | | | + | | | | | | |
| 66A | | + | + | | | | | | |
| 66B | | | | | | | + | | |
| 67A | | | + | + | + | + | | | |
| 67B | | | + | | | | | | |
| 70A | | + | | | | + | | | |
| 70B | | + | | | | | | | |
| 71B | | | | + | | | | | |
| 72A | | | + | | | | | | |
| 72B | | | + | | | | | | |
| 73A | | | + | | | | | | |
| 74B | | | + | | + | | | | |
| 76A | | + | | | | + | | | |
| 78A | | | | + | | | | | |
| 79B | | + | | | | | + | | |
| 81A | | + | | | | | | | |
| 83B | | | | | | | + | | |
| 84A | | | + | | | | | | |
| 85A | | | + | | | | | | |

| Intermediate to Compound No (A or B type) | PI | PV | EG | PO | PS | BC | VI | LN | PH |
|---|---|---|---|---|---|---|---|---|---|
| 86A | | | + | | | | | | |
| 86B | | | | | | | + | | |
| 94B | | + | + | | | | + | | |
| 96B | | + | | | + | | + | | |
| 98B | | + | + | | | | + | | |
| 99A | + | + | + | | | | | + | |
| 106A | | | + | | | + | | | |
| 107A | | + | | | | + | | | |
| 107B | | + | | + | | + | | | |
| 109A | | + | | | | | | | |
| 118A | | | | | | | + | | |
| 118B | | | | | | + | + | | |
| 122A | | + | | | | | | | |
| 122B | | + | | | | | | | |
| 123B | | + | | | | | + | | |
| 124A | | | + | | | + | | | |
| 127A | | + | | | | | | | |
| 127B | | + | | | | | + | | |
| 130A | | + | | | | + | | | |
| 130B | | + | | | | + | | | |
| 133B | | | | | | | + | | |
| 135A | | + | + | | | | | | |
| 136B | | + | | | | + | + | | |
| 137A | | + | | | | + | | | |
| 138B | | | + | | | | + | | |
| 139A | | + | + | | | | + | | |
| 140B | | + | | + | | | | | |
| 141A | | + | | | | + | + | | |
| 141B | | | + | | | | + | | |
| 143A | | + | | | | + | + | | |

| Intermediate to Compound No (A or B type) | PI | PV | EG | PO | PS | BC | VI | LN | PH |
|---|---|---|---|---|---|---|---|---|---|
| 143B | | + | | | | | | + | |
| 144A | | + | | | | + | + | | |
| 145A | | | | | | | + | + | |
| 145B | | | | + | | | | | |
| 146A | | | + | | | | + | | |
| 146B | | | | | | | + | + | |
| 147A | | | | | | + | + | | |
| 147B | | | | | | | + | | + |
| 149A | | | | | | | + | | |
| 150A | | | | | | | + | | |
| 150B | | + | | | | | | | |
| 152A | | + | | | | | | | |
| 152B | | | | | | | + | | |
| 153A | | | | | | | + | | |
| 153B | | | | | | | + | | |
| 154A | | + | | | | | + | | |
| 154B | | + | | | | | + | | |
| 157A | | + | | | | | | | |
| 157B | | + | | | | + | | | |
| 158A | | + | | | | + | | | |
| 160B | | | | | + | | | | |
| 161B | | | | | | | + | | |
| 168A | | + | | + | | | | | |
| 168B | | + | | | | | | + | |
| 169A | | + | | | | | + | | |
| 169B | | + | | | | + | | | |
| 170B | | + | | | | | | | |
| 171A | | + | | | | | | + | |
| 171B | | + | | + | | | + | + | |
| 175A | | | | | | | + | | |

| Intermediate to Compound No (A or B type) | PI | PV | EG | PO | PS | BC | VI | LN | PH |
|---|---|---|---|---|---|---|---|---|---|
| 181B | | | | | | | + | | |
| 184B | | + | | | | | | | |
| 188B | | + | | | | | | | |
| 189B | | | | | | | + | | |
| 192B | | | | + | | | | | |
| 194A | | + | | | | | + | + | |
| 195B | | + | + | | | + | | | |
| 198A | | | + | | | | | | |
| 199A | | + | | | | | | | |
| 199B | | + | | | | | | | |
| 201A | | + | | | | | | | |
| 201B | + | + | | | | | | | |
| 207A | | | | + | | | | | |
| 211B | | | + | | | | + | | |
| 212A | | + | | | | | | | |
| 212B | | + | | | | | | | |
| 216A | | + | | | | | + | | |
| 219A | | | + | | | | | | |
| 219B | | | + | | | | + | | |
| 221A | | | | | | | + | | |
| 223A | | | | | | | | + | |
| 223B | | | | | | | + | + | |
| 227A | | | + | | | | | | |
| 227B | | | | | | | | + | |
| 230B | | + | | | | | | | |
| 234A | | | | + | | | | | |
| 238B | | | | | | | + | | |
| 265A | | + | | | | | + | | |
| 266A | | + | | | | | | | |
| 266B | | + | | | | + | | | |

## Claims

**1.** A compound of formula I

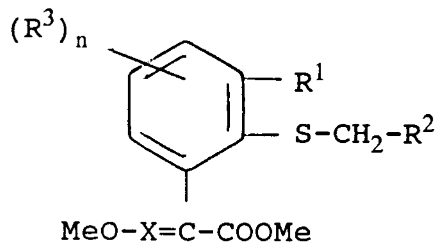

(I)

wherein

| | |
|---|---|
| X | is CH or N; |
| n | is 0, 1, 2 or 3; |
| $R^1$ and $R^3$, | which may be the same or different, are alkyl, alkoxy or alkylthio, each of which is optionally substituted, halogen, nitro, cyano, $COOR^4$, $-NR^5R^6$,$CONR^5R^6$, $COR^7$ or $R^8S(O)_q$; or $R^1$ and an adjacent $R^3$ group, or two adjacent $R^3$ groups, together with the carbon atoms to which they are attached can form a 5 to 8 membered ring which can include 1 to 3 heteroatoms and may be substituted; |
| $R^2$ | is an optionally substituted aliphatic hydrocarbon radical, which may be unsaturated, aryl or heterocyclyl; |
| $R^4$ | is hydrogen or an ester forming group; |
| $R^5$ and $R^6$ | are the same or different and are hydrogen, optionally substituted alkyl, acyl or aryl, or together with the nitrogen to which they are attached, form a 5 to 7 membered ring which can contain other hetero atoms; |
| $R^7$ | is hydrogen, optionally substituted alkyl or aryl; |
| $R^8$ | is optionally substituted alkyl or aryl; and |
| q | is 1 or 2. |

**2.** A compound of formula II

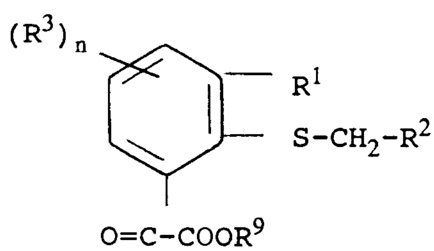

(II)

wherein $R^1$, $R^2$, $R^3$ and n have the meanings given in claim 1 and $R^9$ is hydrogen or methyl.

**3.** A compound of formula III

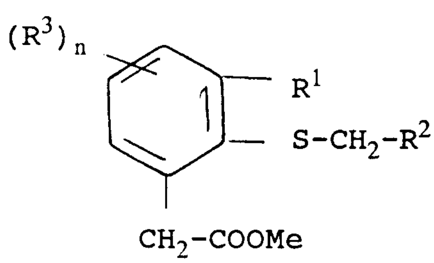

(III)

wherein $R^1$, $R^2$, $R^3$ and n have the meanings given in claim 1.

**4.** A fungicidal composition comprising a compound as claimed claim 1, in admixture with an agriculturally acceptable diluent or carrier.

5. A method of combating phytopathogenic fungi which comprises applying to the fungus or its locus a compound claimed in claims 1.

European Patent Office

# EUROPEAN SEARCH REPORT

Application Number

EP 92 20 1504

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | EP-A-0 299 694 (SCHERING AGROCHEMICALS LTD)<br>* Pages 14-15; claims 1,3,4 *<br>--- | 1,4,5 | C 07 C 323/62<br>A 01 N 37/38<br>C 07 C 323/63<br>C 07 C 323/65<br>C 07 D 239/26<br>C 07 D 249/08<br>C 07 D 271/06<br>C 07 D 277/10<br>C 07 D 277/26<br>A 01 N 37/50<br>A 01 N 43/00 |
| D,X | EP-A-0 251 082 (BASF AG)<br>* Pages 17-18; claims 1-3 *<br>--- | 1,5 | |
| X | EP-A-0 254 426 (IMPERIAL CHEMICAL INDUSTRIES PLC)<br>* Pages 31-32; claims 1,2,7,8 *<br>--- | 1,4,5 | |
| P,X | WO-A-9 203 411 (SCHERING AGROCHEMICALS LTD)<br>* Pages 15-16; claims 1-4 *<br>----- | 2 | |

TECHNICAL FIELDS SEARCHED (Int. Cl.5)

C 07 C

The present search report has been drawn up for all claims

| Place of search | Date of completion of the search | Examiner |
|---|---|---|
| THE HAGUE | 26-08-1992 | FINK D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)